# EUROPEAN PATENT SPECIFICATION

(11) **EP 2 408 350 B1**
(45) Date of publication and mention of the grant of the patent: **13.09.2023**
(21) Application number: 10753760.7
(22) Date of filing: 15.03.2010
(51) Int. Cl.: G01G 19/414, G01G 23/37, G01G 23/36, G16H 20/60, G16H 40/67, G06Q 50/22, G09B 19/00, G09B 23/28, A61B 5/00

(54) **METHOD AND SYSTEM FOR MEASURING AND PRESENTING EATING RATE**
VERFAHREN UND VORRICHTUNG ZUR MESSUNG UND DARSTELLUNG VON ESS-RATEN
PROCÉDÉ ET SYSTÈME DE MESURE ET DE PRÉSENTATION DE TAUX D'ALIMENTATION

(30) Priority: 19.03.2009 SE 0900352; 23.03.2009 US 409364
(43) Date of publication of application: 25.01.2012
(73) Proprietor: Mandometer AB, 141 04 Huddinge (SE)
(72) Inventor: BERGH, Cecilia, S-118 20 Stockholm (SE); SÖDERSTEN, Per, S-118 20 Stockholm (SE)
(74) Representative: Bjerkén Hynell KB
(86) International application number: PCT/SE2010/000061
(87) International publication number: WO 2010/107361

(56) References cited:
- WO-A1-01/39089
- WO-A1-96/02183
- WO-A1-2005/002430
- WO-A2-02/25228
- US-A- 5 233 520
- US-A- 5 817 006
- US-A1- 2002 099 274
- US-A1- 2002 124 017
- US-A1- 2007 073 178
- US-B1- 6 790 178
- ZANDIAN M ET AL: "Decelerated and linear eaters: Effect of eating rate on food intake and satiety", PHYSIOLOGY AND BEHAVIOR, ELSEVIER SCIENCE LTD., OXFORD, GB, vol. 96, no. 2, 16 February 2009 (2009-02-16), pages 270-275, XP025872946, ISSN: 0031-9384, DOI: 10.1016/J.PHYSBEH.2008.10.011 [retrieved on 2008-10-18]
- MODJTABA ET AL.: 'Decelerated and linear eaters: effect of eating rate on food intake and satiety' PHYSIOLOGY AND BEHAVIOUR, US vol. 96, no. 2, 16 February 2009, pages 270 - 275, XP025872946
- WESTERTERP-PLATENGA ET AL.: 'The shape of the cumulative food intake curve in humans, during basic and manipulated meals' PHYSIOLOGY AND BEHAVIOR, US vol. 47, no. 3, 1990, pages 569 - 576, XP023967239
- LINDGREN ET AL.: 'Eating behavior in Prader-Willi syndrome, normal weight, and obese control groups' JOURNAL OF PEDIATRICS, US vol. 137, no. 1, 2000, pages 50 - 55, XP003025670

## Description

### Technical field

The present invention pertains to a system comprising a portable medical apparatus to measure an eating rate utilized to teach persons to eat according to a predetermined graphic eating curve displayed on a screen, and a method therefore.

### Background art

An apparatus named "Mandometer^{®}" has been developed at the Section of Applied Neuroendocrinology and Mandometer^{®} Clinic, Karolinska Institutet, Stockholm, Sweden. It consists of a scale that is connected to a computer. A plate is placed on the scale, the patient puts a measured portion of food determined by a therapist on the plate and the computer records and stores the weight loss from the plate while the patient eats.

This yields a curve of eating rate which is visible to the patient on the computer screen during a meal and can be compared to a pre-set eating curve on screen. At regular intervals, a rating scale appears on the monitor of the computer and the patient rates her/his level of fullness/satiety. The scale has numerical values from 0 (no satiety) to 100 (maximum satiety). As the patients rate their satiety a dot appears on the screen and yields a curve of the development of satiety (fullness). The patients can thus compare their development of fullness to a "normal" fullness curve again pre-set on screen. During "Mandometer^{®}-training" the patient gradually adopts a more normal pattern of eating and satiety by following the training curves, which are displayed on the monitor during the meal. These methods were originally developed for treating eating disorders such as anorexia and bulimia nervosa: they have been evaluated in a randomized controlled trial with an estimated rate of remission of 75%. It was suggested many years ago that obese people eat at an increased rate and in a pilot study on obese adolescents using Mandometer^{®} this observation was confirmed.

The apparatus Mandometer^{®} is patented in several countries for instance in the U.S. under the Patent No. US5817006 to Bergh et al.

Further prior art referring to this technical field can be found in document Zandian M. et al., "Decelerated and linear eaters: Effect of eating rate on food intake and satiety", in Physiology and behavior, Elsevier Science Ltd., Oxford, GB, vol. 96, no. 2, 16 February 2009, pages 270-275, XP025872946, ISSN: 0031-9384, in which a study is disclosed that compares the eating behavior of women with respect to linear and non-linear eating curves.

Further prior art referring to this technical field can be found in document WO 96/03293, disclosing a method and a device for the measurement of rate of eating of an object of measurement ingesting food. The method includes a scale for the measurement of food intake and a first means, which is signal connected with the scale and allows storing and computing of signals and visualization of stored and computed signals. Simultaneously, said first means provides at least one reference standard for rate of eating and an associated, biologically determined level of satiety, which is used for comparison, visualization and evaluation together with the values of rate of eating recorded by said scale and stored by said first means. This allows for adjustment of rate of eating. Said first means of the method consists of a computer system, specifically a portable computer with an integrated scale.

### Summary of the invention

The present invention has as one aim to provide a portable apparatus, a portable medical apparatus and a method for using an apparatus measuring eating rate utilized to teach persons to eat in a normal eating rate in order to aid them to feel full after an intake of a meal, and still gaining or losing weight in a controlled scientific environment. Another aim is to make possible for those persons to be able to eat at any location that has cellular phone coverage, thereby being able to connect to a remote main computer/server at any chosen time to record their eating behavior to be evaluated by experts/therapist in that field. The experts can also provide those using the apparatus with suggestions of which reference standard to utilize depending on the measurements results sent to the main computer/server.

Resent scientific studies have shown that a descending rate of eating prevent persons to overeat by eating in a correct manor which has clinical implication for obese to lose weight.

As such the present invention sets forth a system comprising a portable medical apparatus according to independent claim 1 and a method of using the same according to independent method claim 9.

Further advantageous features are defined in the dependent claims.

### Brief description of the drawings

Henceforth reference is had to the attached figures in the accompanying text of the description for a better understanding of the present invention with its embodiments and given examples, wherein:
**Fig. 1** is illustrating graphics for a prior art linear eating behaviour;
**Fig. 2** is illustrating graphics for a descending eating behaviour in accordance with the present invention;
**Fig. 3** is schematically illustrating an apparatus with cellular radio capabilities in contact with a scale through near field radiation communication in accordance with the present invention;
**Fig. 4** is schematically illustrating another embodiment of an apparatus with cellular radio capabilities, placed in a docking station of a scale, and in contact with the scale through near field radiation communication in accordance with the present invention; and
**Fig. 5** is schematically illustrating another embodiment of an apparatus with cellular radio capabilities, placed in a docking station of a scale through a joint/hinge, and in contact with the scale through near field radiation communication in accordance with the present invention.

### Detailed description of preferred embodiments

The present invention is related to a system and a method to a preferably handheld portable medical measuring apparatus consisting of a first device with cellular phone capabilities such as cellular phones, PDA's and like devices that are handheld as for instance an IPhone ^{®} with a suitable applet adapted to the present invention or other suitable software, and a second device in form of a scale for measuring every portion of food eaten by a person from a plate or bowl or the like placed on the scale during a meal. This apparatus is utilized in teaching persons suffering from anorexia nervosa, bulimia nervosa, obesity, other gastrointestinal problems and persons that would like to have control of their intake of food to keep them fit.

Such a portable device with cellular phone capabilities can be helpful in several situations. It can for instance be utilized by a person to keep arbitrary contact with an expert! therapist for receiving e.g. advices, comfort, and to immediately transmit eating patterns or at least at one of a predetermined time, at the will of a person utilizing the medical apparatus, at the finishing of at least one session and at an arbitrary time. The expert/therapist can also provide those using the apparatus with suggestions of which reference standard to utilize when eating depending on the measurements results sent to the main computer/server. Hence, a person eating can be advised how to adapt the eating behavior compared with a previous registered measurement to improve/adapt the eating behavior.

A medical apparatus according to the present invention and its technical field is unique as it creates total freedom for persons utilizing it, i.e. they do not need to sit at a medical institution or at home. Now they can eat with the apparatus at almost any given place and still be able to receive response to their eating behavior from an expert at a remote site. It is also of outermost importance that the person eating has a small pocket size apparatus, which can be utilized at a discrete site thus avoiding feeling ashamed and having other persons glance at them, which will be the case with previous bigger apparatuses in the present technical field. Such bigger devices are more suitable in an institution or at home.

That people feel uncomfortable or ashamed when eating with a special apparatus which is visible to others has been observed during clinical trials and does not promote a positive feeling to people who are already stigmatized.

A study regarding obesity among children and youth was conducted at Bristol Royal Hospital for Children in England utilizing the "Mandometer^{®}" (prior art), which was developed at the Section of Applied Neuroendocrinology and Mandometer^{®} Clinic, Karolinska Institutet, Stockholm, Sweden. It consists of a scale that is connected to a computer. A plate is placed on the scale; the patient puts a measured portion of food determined by a therapist on the plate and the computer records and stores the weight loss from the plate while the patient eats.

This yields a curve of eating rate which is visible to the patient on the computer screen during their meal and can be compared to a pre-set eating curve or reference standard on screen.

At regular intervals, a rating scale appears on the monitor of the computer and the patient rates her/his level of fullness. The scale has for instance numerical values from 0 (no satiety) to 100 (maximum satiety). As the patient rates their satiety a dot appears on the screen and yields a curve of the development of satiety (fullness). The patient can thus compare their development of fullness to a "normal" fullness curve again pre-set on screen. During "Mandometer^{®}-training" the patient gradually adopts a more normal pattern of eating and satiety by following the training curves, which are displayed on the monitor during the meal. These methods were originally developed for treating eating disorders such as anorexia and bulimia nervosa.

This study came to a further improved possible outcome, namely that Mandometer^{®} curves for practicing eating should probably have a decelerated shape, as this pattern may protect individuals for overeating. Analysis of the average speed of eating, as used here, neglects minute-to-minute changes during the meal, which may explain the absence of a statistically significant effect on this simplified measure of speed of eating.

The findings of decelerated eating have been undergoing further scientific tests and analysis emanating in the following scientific papers "Decelerated and linear eaters: Effect of eating rate on food intake and satiety Modjtaba Zandian a, loannis loakimidis a, Cecilia Bergh a, Ulf Brodin a,b, Per Södersten a, published by Elsevier, Physiology & Behavior, received March 18, 2008, received in revised form 2 October 2, 2008, and accepted October 9, 2008:
a) Karolinska Institutet, Section of Applied Neuroendocrinology, NVS, and Mandometer and Mandolean Clinics, AB Mando, Novum, S-141 57 Huddinge, Sweden
b) Karolinska Institutet, LIME, Section of Medical Statistics, S-171 77 Stockholm, Sweden.

Another published paper is "Linear eaters turned decelerated: Reduction of a risk for disordered eating?" to Modjtaba Zandian, loannis loakimidis, Cecilia Bergh, Per Södersten, published by Elsevier, Physiology & Behavior, received July 4, 2008, revised November 14, 2008, and accepted November 25, 2008.

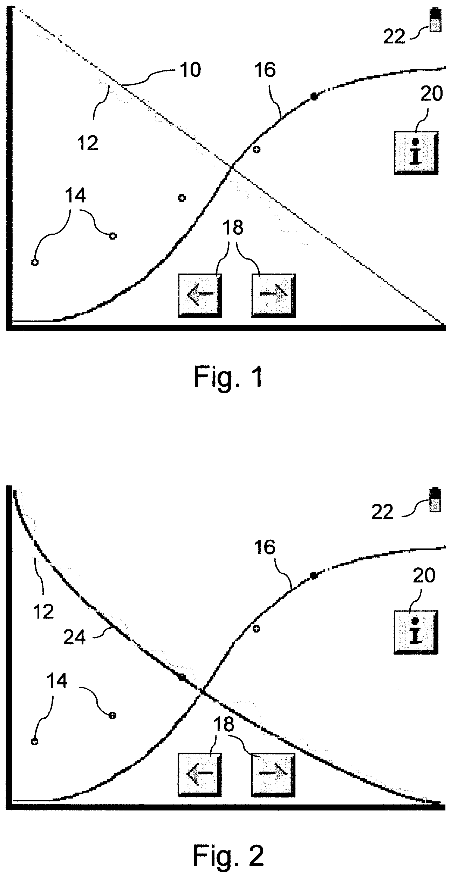

Henceforth, the apparatus utilized for the descended eating rate scientific findings is described. In Fig. 1 graphics are illustrated for prior art linear eating, which was the prior standard for measuring eating rate and satiety utilizing the Mandometer^{®} measuring device or the apparatus of the present invention. Hereby, a predetermined linear eating rate curve 10 is depicted together with an actual real time eating curve 12 recorded from a person utilizing the Mandometer^{®} measuring device, or the measuring device of the present invention. The curve 12 should as much as possible match the linear curve/reference standard 10 to achieve an acceptable normal eating rate, which can aid the person eating to gain or lose weight depending on the persons physical condition. It is appreciated that other reference standard curves can be utilized then those shown in Fig. 1 and Fig. 2.

The dots 14 show how the person eating has rated the satiety during predetermined time periods/intervals, which if connected with a line make up a curve itself illustrating the satiety/fullness of the person eating. Moreover, the Fig. 1 depicts a predetermined sigmoid curve 16 mimicking a normal satiety rating to be compared with the satiety ratings 14 made by the person eating. Buttons 18 are touch buttons utilized to browse between different stored meals in the device with cellular capabilities in accordance with the present invention. Also shown in Fig. 1 is an information button 20, which if pushed for instance shows the amount of food eaten during a session and the duration of the session. Also shown is an icon for the battery charge 22 of the cellular device.

Fig. 2 illustrates similar curves and functions as in Fig. 1, but with the difference that a curve 24 shows a descended eating rate according to recent scientific research results, which better mimics an average persons eating rate.

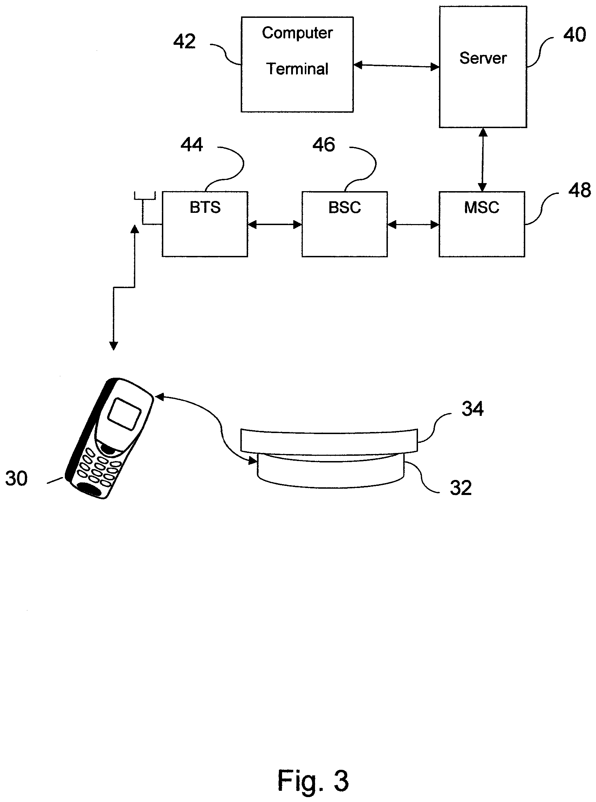

Fig. 3 schematically depicts an embodiment of the system apparatus according to the present invention by comprising a cellular phone 30 with a display screen of any known constitution such as having a conventional keypad or a touch screen. Moreover the apparatus comprises a scale 32 of preferably a small pocket size. It also shows a plate or bowl 34 utilized to put food on when a person is eating. The cellular device 30 and scale 32 communicate with each other through near field radiation such as for instance Bluetooth or Infrared (IR) or the like, schematically depicted through the double arrow.

A communication between the scale and the cellular device 30 transmits data of how much food the person eating removes from the plate 34 in eating intervals, and the eating rate. The person also rates the satiety 14 in predetermined time intervals on the cellular device screen. Hence, the curves for eating rate 12 and satiety 14 according to Fig. 1 and Fig. 2 are displayed so that a person eating from the plate 34 can adapt its eating rate to the curves 10, 24 for a predetermined eating rate and/or compare its satiety to the curves 16 showing satiety.

Moreover, a software means is utilized to transmit the stored session through the first device 30 cellular phone capabilities to a remote server 40 connected with a computer terminal 42 for registration of curves for eating rate 12. A means in the server 40 is able to send adapting instructions to the first device 30 regarding what predetermined graphic eating curve 10, 24 to be utilized from arbitrary geographical places due to previous registrations of real time graphic eating curves in order to adapt the registered eating behaviour of a person utilizing the system apparatus of the present invention.

The cellular phone 30 and server 40 with terminal 32 communicate through known techniques by for instance a base station transceiver (BST) 44 via a base station controller (BSC) 46 and a mobile services switch 48. The communication between devices 40, 42, 44, 46, 48 and cellular phone 30 with scale 32 is depicted through double pointed arrows in Fig. 3.

Persons utilizing the present invention apparatus could in a worst case scenario be in immediate need of medical care, thus it is provided in one embodiment that the location of a person utilizing the apparatus can be tracked through for instance a global positioning system (GPS) residing in the cellular phone 30. Another possibility of tracking the person could be achieved by utilizing the signalling system of the phone 30, which includes wireless mobile location data in the call signal. Wireless mobile location data pin points the real time geographical location of the phone 30, and in most cases also where the user of the phone 30 is located.

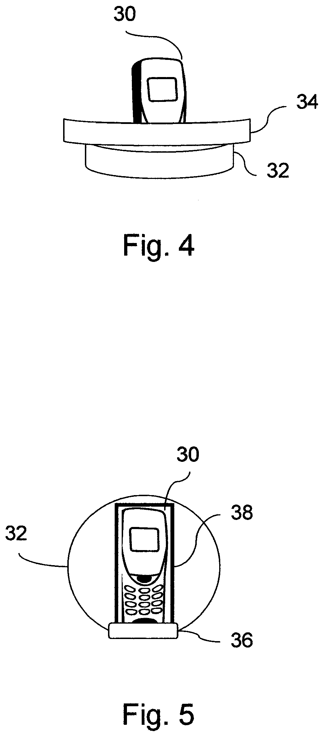

Fig. 4 schematically shows an embodiment of the present invention where the cellular device 30 is docked in a docking station or integrated with the scale 32, it is also showing a plate or bowl 34.

In Fig. 5 it is schematically illustrated an embodiment of a cellular device 30 docked in accordance with Fig. 4 to a scale 32 and folded into a slot 38 in the scale 32 by the aid of a joint/hinge 36. Hereby, the cellular device 30 and the scale form a compact unit when not in use making it easy to keep for instance in a pocket. When in use the cellular device 30 can be folded in at least somewhere between 270 degrees. The docking joint for the cellular device 30 can also be ball like shaped an attached in a ball bearing (not shown), whereby the cellular device can be rotated 360 degrees around its own axes.

The present invention is not restricted to the examples and given embodiments presented above. A person skilled in the art is able to derive further possible embodiments by the attached set of claims.

## Claims

1. A system comprising a portable medical apparatus (30, 32) made up of at least two devices adapted to measure eating rate (12) utilized to teach persons to eat according to a predetermined graphic eating curve (10, 24) displayed on a screen, wherein it comprises:
a first pocket portable device (30) with a screen storing said predetermined graphic eating curve (10, 24) in a memory, said first device (30) having cellular phone capability and remote near field radiation capabilities for communication with a related second device (32);
said related second device (32) being a portable scale adapted to communicate with said first device (30), having cellular phone capability through similar remote near field radiation communication capabilities as said first device (30);
and the system is adapted, during the duration of a scale measurement session, to weigh food on said scale (32), and transmit the food weight to said first device (30) through said near field radiation when food is removed from said scale by a person eating the food, and through means of software residing in said first device (30) to store the amount of food removed during a session in said memory, to display the weight of food removed as a measurement point on said screen when it is removed from said scale to make up a real time graphic eating curve (12) to be compared with said predetermined eating curve (10, 24); and
the system further comprises a software means adapted to transmit said stored session through said first device (30) cellular phone capabilities through a cellular signalling system (44, 46, 48) to a remote server (40) for registration, and a means in said server (40) being able to send adapting instructions to said first device (30) regarding what predetermined graphic eating curve (10, 24) to be utilized from arbitrary places due to previous registrations of real time graphic eating curves **characterized in that**.
said scale (32) has a docking station for said first device (30), whereby at least the screen is visible to a person eating from said scale.

2. A system according to claim 1, wherein said docking station is equipped with a joint/hinge (36) having an adapter to receive said first device having cellular phone capability, said joint/hinge (36)
being adapted to move said first device (30) in a predetermined number of degrees around said first device center axes and/or between different elevation levels.

3. A system according to claim 2, wherein said joint/hinge (36) is utilized to put said first cellular device in a slot/pocket (38) on the back of said scale when not utilized.

4. A system according to claim 1, wherein said scale (32) has its scale measuring surface formed as a bowl or plate to be served food on.

5. A system according to claim 1, wherein said predetermined graphic curve (24) describes a non linear eating behaviour, which mimics an eating behaviour that is favourable to human beings.

6. A system according to claim 1, wherein said predetermined graphic curve (24) describes a non linear descending rate of eating behaviour.

7. A system according to claim 1, adapted to be able to display a predetermined graphic curve of satiety (16) on said screen, for comparison with satiety ratings (14) which can be made during pre-set time intervals on said screen by a person utilizing said apparatus (30, 32).

8. A system according to claim 1, adapted to be able to locate the position of a person utilizing said apparatus (30, 32) through a global positioning system, or by utilizing the signalling system of the phone (30), which includes wireless mobile location data in a call signal.

9. A method utilizing a system comprising a portable medical apparatus (30, 32) in accordance with claim 1 wherein said system is made up of at least two devices adapted to measure eating rate (12) utilized to teach persons to eat according to a predetermined graphic eating curve (10, 24) displayed on a screen, wherein it comprises:
utilizing a first pocket portable device (30) with a screen storing said predetermined graphic eating curve (10, 24) in a memory, said first device (30) having cellular phone capability and remote near field radiation capabilities for communication with a related second device (32);
said related second device (32) being a utilized portable scale adapted to communicate with said first device (30), having cellular phone capability through similar remote near field radiation communication capabilities as said first device (30);
measuring during a scale measurement session the weight of food on said scale (32), and transmitting the food weight to said first device (30) through said near field radiation when food is removed from said scale by a person eating the food, and through means of software residing in said first device (30) storing the amount of food removed during a session in said memory, displaying the weight of food removed as a measurement point on said screen when it is removed from said scale to make up a real time graphic eating curve (12) to be compared with said predetermined eating curve (10, 24); and
utilizing a software means to transmit said stored session through said first device (30) cellular phone capabilities through a cellular signalling system (44, 46, 48) to a remote server (40) for registration, and a means in said server (40) being able to send adapting instructions to said first device (30) regarding what predetermined graphic eating curve (10, 24) to be utilized from arbitrary places due to previous registrations of real time graphic eating curves.

10. A method according to claim 9, wherein a person utilizing said apparatus (30, 32) is located through a global positioning system, or by utilizing the signalling system of the phone (30), which includes wireless mobile location data in a call signal.

## Patentansprüche

1. System, das ein tragbares medizinisches Gerät (30, 32) umfasst, das folgende Teile umfasst:
mindestens zwei Vorrichtungen zur Messung der Essgeschwindigkeit (12), die dazu verwendet werden, Personen zu lehren, nach einer vorgegebenen grafischen Esskurve (10, 24) zu essen, die auf einem Bildschirm angezeigt wird,
wobei es umfasst:
eine erste tragbare Taschenvorrichtung (30) mit einem Bildschirm, auf dem die vorbestimmte graphische Esskurve (10, 24) in einem Speicher gespeichert ist, wobei die erste Vorrichtung (30) eine Mobiltelefon-Fähigkeit und eine Nahfeld-Kommunikations-Strahlungsfähigkeit mit einer zugehörigen zweiten Vorrichtung (32) aufweist;
wobei das zugehörige zweite Gerät (32) eine tragbare Waage ist, die zur Kommunikation mit dem ersten Gerät (30), das über ähnliche Nahfeld-Kommunikations-Strahlungsfähigkeit wie das erste Gerät (30) zellular telefonieren kann;
und das System so ausgelegt ist, dass es während der Dauer einer Waagenmesssitzung Lebensmittel auf der Waage (32) wiegt und das Gewicht der Lebensmittel durch die Nahfeldstrahlung an die erste Vorrichtung (30) überträgt, wenn die Lebensmittel von der Waage durch eine Person, die die Lebensmittel isst, entfernt werden, und mittels einer Software, die sich in der ersten Vorrichtung (30) befindet, um die Menge der während einer Sitzung entnommenen Nahrung in dem Speicher zu speichern, um das Gewicht der entnommenen Nahrung als einen Messpunkt auf dem Bildschirm anzuzeigen, wenn sie von der Waage entnommen wird, um eine grafische Echtzeit-Esskurve (12) zu erstellen, die mit der vorbestimmten Esskurve (10, 24) verglichen werden soll; und
das System ferner ein Softwaremittel umfasst, das angepasst ist, um die gespeicherte Sitzung durch die Mobiltelefonfähigkeiten der ersten Vorrichtung (30) über ein zellulares Signalsystem (44, 46, 48) an einen entfernten Server (40) zur Registrierung zu übertragen, und ein Mittel in dem Server (40), das in der Lage ist, Anpassungsanweisungen an die erste Vorrichtung (30) zu senden, die sich darauf beziehen, welche vorbestimmte grafische Esskurve (10, 24) aufgrund früherer Registrierungen von grafischen Echtzeit-Esskurven von beliebigen Orten aus verwendet werden soll, **dadurch gekennzeichnet, dass**
die Waage (32) eine Andockstation für das erste Gerät (30) aufweist, wodurch zumindest der Bildschirm für eine Person, die von der Waage isst, sichtbar ist.

2. System nach Anspruch 1, wobei die Dockingstation mit einem Gelenk/Scharnier (36) ausgestattet ist, das einen Adapter zur Aufnahme des ersten Geräts mit Mobiltelefonfähigkeit aufweist, wobei das Gelenk/Scharnier (36) geeignet ist, die erste Vorrichtung (30) in einer vorbestimmten Anzahl von Graden um die Mittelachsen der ersten Vorrichtung und/oder zwischen verschiedenen Höhenniveaus zu bewegen.

3. System nach Anspruch 2, wobei das Gelenk/Scharnier (36) verwendet wird, um das erste zelluläre Gerät in einen Schlitz/eine Tasche (38) auf der Rückseite der Waage zu legen, wenn es nicht verwendet wird.

4. System nach Anspruch 1, **dadurch gekennzeichnet, dass** die Messfläche der Waage (32) die Form einer Schale oder eines Tellers hat, auf dem Lebensmittel serviert werden.

5. System nach Anspruch 1, wobei die vorbestimmte grafische Kurve (24) beschreibt ein nicht lineares Essverhalten, das ein für den Menschen günstiges Essverhalten nachahmt.

6. System nach Anspruch 1, wobei die vorgegebene grafische Kurve (24) eine nicht lineare absteigende Rate des Essverhaltens beschreibt.

7. System nach Anspruch 1, das so beschaffen ist, dass es in der Lage ist, eine vorbestimmte grafische Sättigungskurve (16) auf dem Bildschirm anzuzeigen, um sie mit Sättigungsbewertungen (14) zu vergleichen, die während voreingestellter Zeitintervalle von einer Person, die die Vorrichtung (30, 32) benutzt, auf dem Bildschirm vorgenommen werden können.

8. System nach Anspruch 1, das so beschaffen ist, dass es in der Lage ist, die Position einer Person, die das Gerät (30, 32) benutzt, über ein globales Positionierungssystem oder durch Nutzung des Signalisierungssystems des Telefons (30), das drahtlose mobile Standortdaten in einem Anrufsignal enthält, zu lokalisieren.

9. Verfahren unter Verwendung eines Systems, das ein tragbares medizinisches Gerät (30, 32) nach Anspruch 1 umfasst, wobei das System aus mindestens zwei Vorrichtungen besteht, die dazu geeignet sind, die Essgeschwindigkeit (12) zu messen, die verwendet wird, um Personen zu lehren, gemäß einer vorbestimmten grafischen Esskurve (10, 24) zu essen, die auf einem Bildschirm angezeigt wird, umfassend:
Verwenden eines ersten tragbaren Taschengeräts (30) mit einem Bildschirm, auf dem die vorbestimmte grafische Esskurve (10, 24) in einem Speicher gespeichert ist, wobei das erste Gerät (30) eine Mobiltelefon-Fähigkeit und eine Nahfeld-Kommunikations-Strahlungsfähigkeit mit einem zugehörigen zweiten Gerät (32) aufweist;
wobei das zugehörige zweite Gerät (32) eine benutzte tragbare Waage ist, die mit dem ersten Gerät (30) kommunizieren kann, mit Mobiltelefon-Fähigkeit durch ähnliche Nahfeld-Kommunikations-Strahlungsfähigkeiten wie das erste Gerät (30);
Messen des Gewichts von Lebensmitteln auf der Waage (32) während einer Waagenmesssitzung und Übertragen des Lebensmittelgewichts an die erste Vorrichtung (30) durch die Nahfeldstrahlung, wenn Lebensmittel von der Waage durch eine Person, die die Lebensmittel isst, entfernt werden, und durch eine Software, die sich in der ersten Vorrichtung (30) befindet, die die Menge der während einer Sitzung entfernten Lebensmittel in dem Speicher speichert, Anzeigen des Gewichts der entfernten Lebensmittel als ein Messpunkt auf dem Bildschirm, wenn sie von der Waage entfernt werden, um eine grafische Echtzeit-Esskurve (12) zu bilden, die mit der vorbestimmten Esskurve (10, 24) verglichen werden soll; und
Verwenden eines Softwaremittels, um die gespeicherte Sitzung durch die Mobiltelefonfähigkeiten des ersten Geräts (30) über ein zellulares Signalsystem (44, 46, 48) an einen entfernten Server (40) zur Registrierung zu übertragen, und ein Mittel in dem Server (40), das in der Lage ist, Anpassungsanweisungen an das erste Gerät (30) zu senden, die sich darauf beziehen, welche vorbestimmte grafische Esskurve (10, 24) von beliebigen Orten aufgrund früherer Registrierungen von grafischen Echtzeit-Esskurven verwendet werden soll.

10. Verfahren nach Anspruch 9, wobei eine Person, die die Vorrichtung (30, 32) benutzt, durch ein globales Positionierungssystem oder durch Nutzung des Signalisierungssystems des Telefons (30), das drahtlose mobile Standortdaten in einem Anrufsignal enthält, geortet wird.

## Revendications

1. Système comprenant un appareil médical portable (30, 32) composé d'au moins deux dispositifs adaptés à la mesure de la vitesse d'ingestion (12) utilisés pour apprendre aux personnes à manger selon une courbe graphique d'ingestion prédéterminée (10, 24) affichée sur un écran, où le système comprend :
un premier dispositif portable de poche (30) doté d'un écran stockant ladite courbe graphique d'ingestion prédéterminée (10, 24) dans une mémoire, ledit premier dispositif (30) ayant une capacité de téléphonie cellulaire et des capacités de rayonnement en champ proche à distance pour la communication avec un second dispositif connexe (32) ;
ledit second dispositif connexe (32) étant une balance portable adaptée pour communiquer avec ledit premier dispositif (30), ayant une capacité de téléphonie cellulaire par le biais de capacités de communication à distance par rayonnement en champ proche similaires à celles dudit premier dispositif (30) ;
et le système est adapté, pendant la durée d'une session de mesure de la balance, pour peser les aliments sur ladite balance (32), et transmettre le poids des aliments audit premier dispositif (30) par le biais dudit rayonnement en champ proche lorsque les aliments sont retirés de ladite balance par une personne qui mange les aliments, et par le biais d'un logiciel résidant dans ledit premier dispositif (30) pour stocker dans ladite mémoire la quantité d'aliments retirés au cours d'une session, pour afficher le poids des aliments retirés en tant que point de mesure sur ledit écran lorsqu'ils sont retirés de ladite balance pour constituer une courbe graphique d'ingestion en temps réel (12) à comparer avec ladite courbe d'ingestion prédéterminée (10, 24) ; et
le système comprend en outre un moyen logiciel adapté pour transmettre ladite session stockée à travers les capacités de téléphonie cellulaire dudit premier dispositif (30) à travers un système de signalisation cellulaire (44, 46, 48) à un serveur distant (40) pour enregistrement, et un moyen dans ledit serveur (40) pouvant envoyer des instructions d'adaptation audit premier dispositif (30) concernant quelle courbe graphique d'ingestion prédéterminée (10, 24) doit être utilisée à partir d'endroits arbitraires en raison d'enregistrements précédents de courbes graphiques d'ingestion en temps réel, le système étant **caractérisé en ce que** :
ladite balance (32) comporte une station d'accueil pour ledit premier dispositif (30) de sorte qu'au moins l'écran est visible par une personne mangeant à partir de ladite balance.

2. Système selon la revendication 1, dans lequel ladite station d'accueil est équipée d'une articulation/charnière (36) ayant un adaptateur pour recevoir ledit premier dispositif ayant une capacité de téléphonie cellulaire, ladite articulation/charnière (36) étant adaptée pour déplacer ledit premier dispositif (30) suivant un nombre prédéterminé de degrés autour des axes centraux dudit premier dispositif et/ou entre différents niveaux d'élévation.

3. Système selon la revendication 2, dans lequel ladite articulation/charnière (36) est utilisée pour placer ledit premier dispositif cellulaire dans une fente/poche (38) à l'arrière de ladite balance lorsqu'il n'est pas utilisé.

4. Système selon la revendication 1, dans lequel ladite balance (32) a sa surface de mesure formée par un bol ou une assiette où servir de la nourriture.

5. Système selon la revendication 1, dans lequel ladite courbe graphique prédéterminée (24) décrit un comportement d'ingestion non linéaire, qui imite un comportement d'ingestion favorable aux êtres humains.

6. Système selon la revendication 1, dans lequel ladite courbe graphique prédéterminée (24) décrit une vitesse descendante non linéaire de comportement d'ingestion.

7. Système selon la revendication 1, adapté pour pouvoir afficher une courbe graphique prédéterminée de la satiété (16) sur ledit écran, pour comparaison avec des évaluations de satiété (14) qui peuvent être faites pendant des intervalles de temps prédéfinis sur ledit écran par une personne utilisant ledit appareil (30, 32).

8. Système selon la revendication 1, adapté pour pouvoir localiser la position d'une personne utilisant ledit appareil (30, 32) par l'intermédiaire d'un système de géolocalisation de type GPS, ou en utilisant le système de signalisation du téléphone (30), qui inclut des données de localisation mobile sans fil dans un signal d'appel.

9. Procédé utilisant un système comprenant un appareil médical portable (30, 32) selon la revendication 1, où ledit système est constitué d'au moins deux dispositifs adaptés pour mesurer une vitesse d'ingestion (12) utilisée pour apprendre aux personnes à manger selon une courbe graphique d'ingestion prédéterminée (10, 24) affichée sur un écran, où le procédé comprend les étapes suivantes :
utiliser un premier dispositif portable de poche (30) doté d'un écran stockant ladite courbe graphique d'ingestion prédéterminée (10, 24) dans une mémoire, ledit premier dispositif (30) ayant une capacité de téléphonie cellulaire et des capacités de rayonnement en champ proche à distance pour la communication avec un second dispositif connexe (32) ;
ledit second dispositif (32) étant une balance portable utilisée et adaptée pour communiquer avec ledit premier dispositif (30), ayant une capacité de téléphonie cellulaire par le biais de capacités de communication à distance par rayonnement en champ proche similaires à celles dudit premier dispositif (30) ;
mesurer, au cours d'une session de mesure, le poids des aliments sur ladite balance (32), et transmettre le poids des aliments audit premier dispositif (30) par le biais dudit rayonnement en champ proche lorsque les aliments sont retirés de ladite balance par une personne qui mange les aliments, et par le biais d'un logiciel résidant dans ledit premier dispositif (30) stockant dans ladite mémoire la quantité d'aliments retirés au cours d'une session, afficher le poids des aliments retirés en tant que point de mesure sur ledit écran lorsqu'ils sont retirés de ladite balance pour constituer une courbe graphique d'ingestion en temps réel (12) à comparer avec ladite courbe d'ingestion prédéterminée (10, 24) ; et
utiliser un moyen logiciel pour transmettre ladite session stockée à travers les capacités de téléphonie cellulaire dudit premier dispositif (30) par le biais d'un système de signalisation cellulaire (44, 46, 48) à un serveur distant (40) pour enregistrement, et un moyen dans ledit serveur (40) pouvant envoyer des instructions d'adaptation audit premier dispositif (30) concernant quelle courbe graphique d'ingestion prédéterminée (10, 24) doit être utilisée à partir d'endroits arbitraires en raison d'enregistrements précédents de courbes graphiques d'ingestion en temps réel.

10. Procédé selon la revendication 9, dans lequel une personne utilisant ledit appareil (30, 32) est localisée par l'intermédiaire d'un système de géolocalisation de type GPS, ou en utilisant le système de signalisation du téléphone (30), qui inclut des données de localisation mobile sans fil dans un signal d'appel.
